# EUROPEAN PATENT APPLICATION

(11) **EP 4 811 362 A1**
(43) Date of publication of application: **23.09.2026**
(21) Application number: 24891320.4
(22) Date of filing: 07.11.2024
(51) Int. Cl.: G16B 15/00

(54) **ANTIBODY DYNAMIC STRUCTURAL PROPERTY ANALYSIS DEVICE, ANTIBODY DYNAMIC STRUCTURAL PROPERTY ANALYSIS METHOD, AND PROGRAM**

(30) Priority: 16.11.2023 JP 2023195471
(71) Applicant: National University Corporation Kumamoto University, Kumamoto 860-8555 (JP)
(72) Inventor: MORIOKA Hiroshi, Kumamoto-shi, Kumamoto 860-8555 (JP); KOBASHIGAWA Yoshihiro, Kumamoto-shi, Kumamoto 860-8555 (JP); SATO Takashi, Kumamoto-shi, Kumamoto 860-8555 (JP); OKAZAKI Kyo, Kumamoto-shi, Kumamoto 860-8555 (JP); SUI Yixuan, Kumamoto-shi, Kumamoto 860-8555 (JP)
(74) Representative: Plougmann Vingtoft a/s
(86) International application number: PCT/JP2024/039609
(87) International publication number: WO 2025/105282

(57) **Abstract**

The present invention relates to an antibody dynamic structural property analyzer and other techniques that enable analysis of movements of residues without averaging. In an antibody dynamic structural property analyzer (1), a molecular movement calculator (29) obtains a plurality of snapshots for a target antibody through, for example, molecular dynamics calculation. An R-plot processor (31) generates a Ramachandran plot for an n-th residue in N residues of the target antibody and obtains dihedral angles φ and ψ of the n-th residue in each snapshot. A collective variable processor (35) calculates distances from reference dihedral angle values φ⁰ₙ and ψ⁰ₙ for the n-th residue to obtain a value of a collective variable for the dihedral angles φ and ψ of the n-th residue in each snapshot. A motion analysis processor (39) determines that the n-th residue is a deviating residue when, in collective variables for the n-th residue in the plurality of snapshots, a proportion of collective variables having values in a region exceeding a motion analysis reference value exceeds a reference proportion value.

## Description

### TECHNICAL FIELD

The present invention relates to an antibody dynamic structural property analyzer, an antibody dynamic structural property analysis method, and a program, and more particularly, to, for example, an antibody dynamic structural property analyzer that uses identification data for a target antibody stored in an analysis target storage to analyze the dynamic structural properties of the antibody.

### BACKGROUND

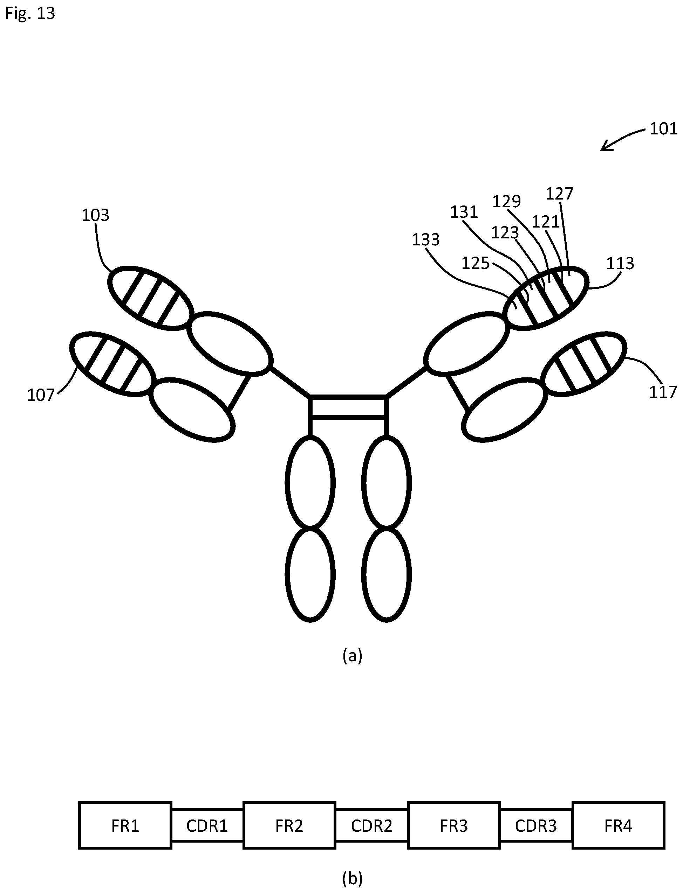

FIGs. 13a and 13b are diagrams each describing an antibody. An antibody is a biopolymer in the immune system that binds specifically to an antigen of a foreign substance (e.g., a pathogen) to remove the foreign substance.

In FIG. 13a, an antibody 101 is one of immunoglobulins, or IgG (150 kDa). The antibody can bind to a specific antigen. Combinations of heavy chain variable regions (H chain variable regions) 103 and 113 and light chain variable regions (L chain variable regions) 107 and 117 diversify the antibody. The H chain variable regions 103 and 113 together with the L chain variable regions 107 and 117 are variable regions of an antigen receptor.

Complementarity determining regions (CDRs) in the variable regions 103, 107, 113, and 117 actually come in contact with an antigen. The CDRs are important regions for determining antigen specificity and have highly diverse sequences. In FIG. 13a, the reference numerals 121, 123, and 125 respectively indicate a CDR 1, a CDR 2, and a CDR 3 in the variable region 113.

Framework regions (FRs) are regions other than CDRs1, CDRs 2, and CDRs 3 in the variable regions 103, 107, 113, and 117. FRs have high sequence homology between antibodies, providing a skeletal structure and stability to an antibody to support the functions of the antibody. In FIG. 13a, the reference numerals 127, 129, 131, and 133 respectively indicate an FR 1, an FR 2, an FR 3, and an FR 4 in the variable region 113.

FIG. 13b shows the relationship of the CDR 1, CDR 2, and CDR 3 and the FR 1, FR 2, FR 3, and FR 4. The CDR 1 is located between the FR 1 and FR 2. The CDR 2 is located between the FR 2 and FR 3. The CDR 3 is located between the FR 3 and FR 4.

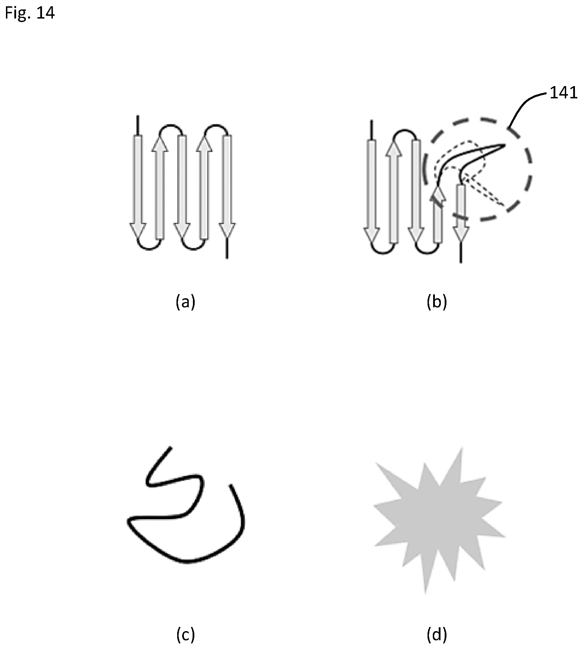

FIGs. 14a to 14d are diagrams each describing a denaturation process of a protein. FIG. 14a shows the protein in a natural state. FIG. 14b shows the protein in a state (locally denatured state) with a locally denatured region (weak spot) 141. The protein is then fully denatured as shown in FIG. 14c. The protein thus forms an aggregate as shown in FIG. 14d.

Stabilizing the locally denatured region 141 in FIG. 14b improves stability of the protein. The locally denatured region can be found using root mean square fluctuation (RMSF) values. RMSF values are indicators of fluctuation obtained with a molecular dynamics method. The inventors have proposed, in Non-Patent Literature 1, that a stable antibody can be designed by finding a locally denatured region using RMSF values as indicators.

### CITATION LIST

### NON-PATENT LITERATURE

Non-Patent Literature 1: Okazaki K and eight other authors, Molecular Dynamics-Based Design and Biophysical Evaluation of Thermostable Single-Chain Fv Antibody Mutants Derived from Pharmaceutical Antibodies. ACS Omega, 2023, 8, 22945-22954.

### SUMMARY OF INVENTION

### TECHNICAL PROBLEM

Analysis using RMSF values relies on manual analysis and thus has a limit in the number of searchable variants. Additionally, residue movements are averaged in RMSF values. Rare movements or small movements are thus not easily evaluated.

One or more aspects of the present invention are directed to an antibody dynamic structural property analyzer that can analyze movements of a residue without averaging the movements.

### SOLUTION TO PROBLEM

An antibody dynamic structural property analyzer according to a first aspect of the present invention is an analyzer for analyzing dynamic structural properties of a target antibody using identification data for the target antibody stored in an analysis target storage. The analyzer includes a molecular movement calculator that calculates movements of at least atoms or molecules and obtains a plurality of snapshots for the target antibody, an R-plot processor that generates a Ramachandran plot for an n-th residue (n is a natural number from 1 to N) in N residues (N is a natural number) of the target antibody and obtains dihedral angles φ and ψ of the n-th residue in each of the plurality of snapshots, a collective variable processor that calculates distances from reference dihedral angle values φ⁰ₙ and ψ⁰ₙ for the n-th residue to obtain a value of a collective variable for the dihedral angles φ and ψ of the n-th residue in each of the plurality of snapshots, and a motion analysis processor that determines that the n-th residue is a deviating residue when, in collective variables for the n-th residue in the plurality of snapshots, a proportion of collective variables having values in a region exceeding a motion analysis reference value exceeds a reference proportion value.

An antibody dynamic structural property analyzer according to a second aspect of the present invention is the analyzer according to the first aspect further including a mutation processor that stores, into the analysis target storage, identification data for another target antibody resulting from introduction of a mutation into the target antibody.

An antibody dynamic structural property analyzer according to a third aspect of the present invention is the analyzer according to the first aspect or the second aspect further including an R-plot analysis reference value determiner. The molecular movement calculator calculates movements of at least atoms or molecules and obtains a plurality of snapshots for a plurality of reference antibodies. The R-plot processor generates a Ramachandran plot for n-th residues of the plurality of reference antibodies and obtains dihedral angles φ and ψ of each of the n-th residues in the plurality of snapshots. The R-plot analysis reference value determiner determines the reference dihedral angle values φ⁰ₙ and ψ⁰ₙ for the n-th residue using the Ramachandran plot for the n-th residues of the plurality of reference antibodies.

An antibody dynamic structural property analysis method according to a fourth aspect of the present invention is a method for analyzing dynamic structural properties of a target antibody using identification data for the target antibody stored in an analysis target storage. The method includes calculating, with a molecular movement calculator included in an information processing device, movements of at least atoms or molecules and obtaining a plurality of snapshots for the target antibody, generating, with an R-plot processor included in the information processing device, a Ramachandran plot for an n-th (n is a natural number from 1 to N) residue in N residues (N is a natural number) of the target antibody and obtaining dihedral angles φ and ψ for the n-th residue, calculating, with a collective variable processor included in the information processing device, distances from reference dihedral angle values φ⁰ₙ and ψ⁰ₙ to obtain a value of a collective variable for the dihedral angles φ and ψ of the n-th residue in each of the plurality of snapshots, and determining, with a motion analysis processor included in the information processing device, that the n-th residue is a deviating residue when, in collective variables for the n-th residue in the plurality of snapshots, a proportion of collective variables having values in a region exceeding a motion analysis reference value exceeds a reference proportion value.

An antibody dynamic structural property analysis method according to a fifth aspect of the present invention is the method according to the fourth aspect further including storing, into the analysis target storage, with a mutation processor included in the information processing device, identification data for another target antibody resulting from introduction of a mutation into the target antibody.

An antibody dynamic structural property analysis method according to a sixth aspect of the present invention is the method according to the fourth aspect or the fifth aspect further including calculating, with the molecular movement calculator, movements of at least atoms or molecules and obtaining a plurality of snapshots for a plurality of reference antibodies, generating, with the R-plot processor, a Ramachandran plot for n-th residues of the plurality of reference antibodies and obtaining dihedral angles φ and ψ of each of the n-th residues in the plurality of snapshots, and determining, with an R-plot analysis reference value determiner included in the information processing device, the reference dihedral angle values φ⁰ₙ and ψ⁰ₙ for the n-th residue using the Ramachandran plot for the n-th residues of the plurality of reference antibodies.

A program according to a seventh aspect of the present invention is a program for causing a computer to function as the antibody dynamic structural property analyzer according to any one of the first to third aspects. Another aspect of the present invention may be directed to a computer-readable recording medium storing the program according to the seventh aspect.

The n-th residue may be, for example, a residue in a framework region. In other words, the technique in one aspect of the present invention may determine whether the n-th residue is a deviating residue for each n-th residue (n is a natural number from 1 to N) in N residues (N is a natural number) that are some of or all of the residues in the framework region.

In the third aspect or the sixth aspect, the collective variable processor may calculate distances from the reference dihedral angle values φ⁰ₙ and ψ⁰ₙ to obtain a value of a collective variable for the dihedral angles φ and ψ of the n-th residue in each of the snapshots, and the motion analysis processor may perform processing for setting the n-th motion analysis reference value as a reference value for determining that the n-th residue has common movements between antibodies.

In one aspect of the present invention, the motion analysis processor may determine that the n-th residue is a deviating residue when, in collective variables for the n-th residue in the plurality of snapshots, the proportion of collective variables having values in a region exceeding the motion analysis reference value (a region in which the value of the collective variable is greater than the motion analysis reference value) exceeds the reference proportion value (greater than the reference proportion value) and that the n-th residue is not a deviating residue when the proportion does not exceed the reference proportion value (less than or equal to the reference proportion value).

### ADVANTAGEOUS EFFECTS

The technique according to the above aspects of the present invention can achieve analysis without averaging the movements of a residue by analyzing one-dimensional values, or the collective variables, calculated based on two-dimensional values, or the dihedral angles φ and ψ, in a Ramachandran plot for the snapshots obtained by calculating the movements of at least atoms or molecules. The technique can find a locally denatured state that is unfindable in analysis using RMSF values. Multiple antibodies can also be compared at the same time. The technique according to the above aspects of the present invention thus allows rapid designing of antibodies with notably high evaluation performance and high thermal stability. Additionally, more stable antibodies may be produced.

### BRIEF DESCRIPTION OF DRAWINGS

FIG. 1 is a block diagram of an antibody dynamic structural property analyzer 1 according to an embodiment of the present invention, showing an example structure.
FIG. 2 is a flowchart of an example operation of the antibody dynamic structural property analyzer 1 in FIG. 1.
FIG. 3 is a flowchart of an example operation of the antibody dynamic structural property analyzer 1 in FIG. 1.
FIG. 4 is a graph of example collective values for an n-th residue in which the proportion of collective variables in a region exceeding an n-th motion analysis reference value does not exceed a reference proportion value.
FIGs. 5a and 5b are first graphs describing an example experiment conducted by the inventors using trastuzumab antibodies.
FIGs. 6a and 6b are second graphs describing the example experiment conducted by the inventors using trastuzumab antibodies.
FIGs. 7a to 7j are third graphs describing the example experiment conducted by the inventors using trastuzumab antibodies.
FIGs. 8a to 8h are fourth graphs describing the example experiment conducted by the inventors using trastuzumab antibodies.
FIGs. 9a and 9b are first graphs describing an example experiment conducted by the inventors using OKT3 antibodies.
FIGs. 10a and 10b are second graphs describing the example experiment conducted by the inventors using OKT3 antibodies.
FIGs. 11a to 11j are third graphs describing the example experiment conducted by the inventors using OKT3 antibodies.

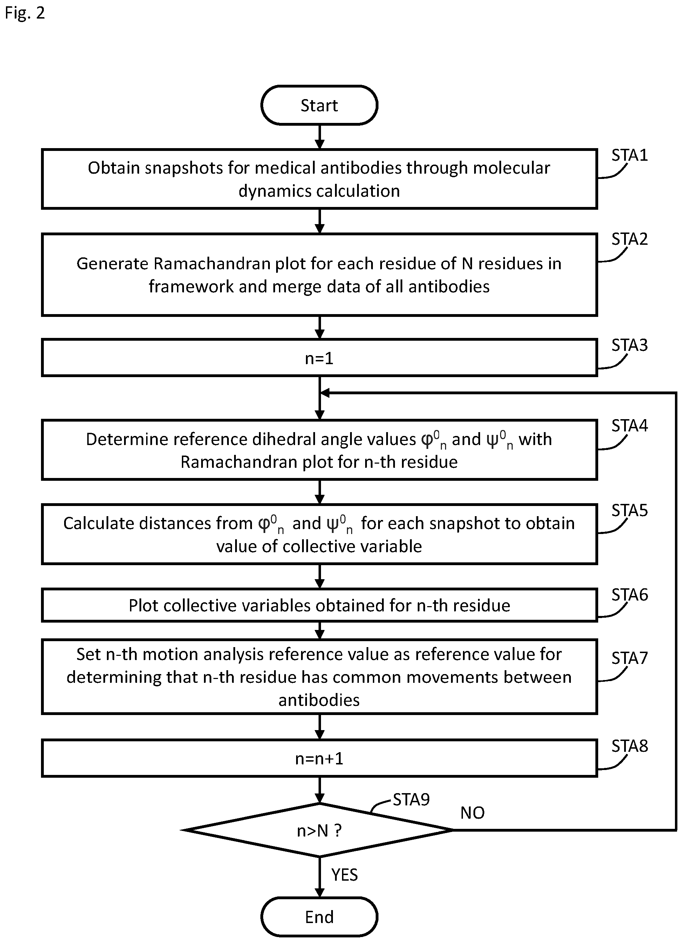

FIGs. 2a to 12j are fourth graphs describing the example experiment conducted by the inventors using OKT3 antibodies.
FIGs. 13a and 13b are diagrams each describing an antibody.
FIGs. 14a to 14d are diagrams each describing a denaturation process of a protein.

### DETAILED DESCRIPTION

An embodiment of the present invention will be described below with reference to the drawings. The present invention is not limited to the embodiment below.

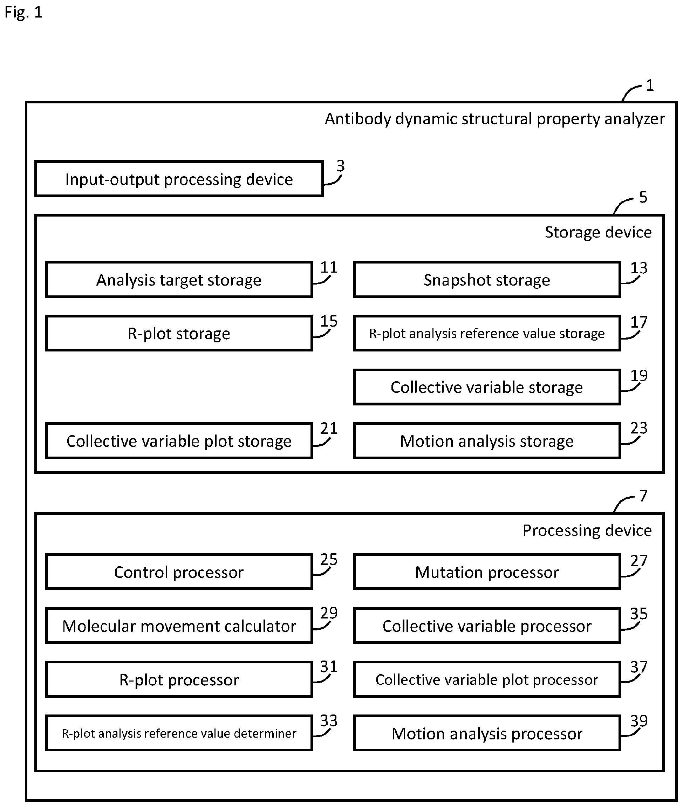

FIG. 1 is a block diagram of an antibody dynamic structural property analyzer 1 according to an embodiment of the present invention, showing an example structure.

The antibody dynamic structural property analyzer 1 is, for example, an information processing device. The antibody dynamic structural property analyzer 1 includes an input-output processing device 3, a storage device 5, and a processing device 7.

The input-output processing device 3 is a device with which data is input from and output to an external person or an external apparatus. The input-output processing device 3 is, for example, a keyboard, a mouse, a display, or a touchscreen with which data is input into the antibody dynamic structural property analyzer 1 by a user or is output to the user. The input-output processing device 3 communicates with, for example, another device to obtain data from, for example, an external information processing device or a database or to transmit data to an external information processing device.

The storage device 5 is a device that stores data, such as a memory or a hard disk. The storage device 5 includes an analysis target storage 11, a snapshot storage 13, an R-plot storage 15, an R-plot analysis reference value storage 17, a collective variable storage 19, a collective variable plot storage 21, and a motion analysis storage 23.

The processing device 7 is, for example, a central processing unit (CPU) and processes data. The processing device 7 includes a control processor 25, a mutation processor 27, a molecular movement calculator 29, an R-plot processor 31, an R-plot analysis reference value determiner 33, a collective variable processor 35, a collective variable plot processor 37, and a motion analysis processor 39. The components of the processing device 7 may be implemented by, for example, the processing device 7 operating under control of a program.

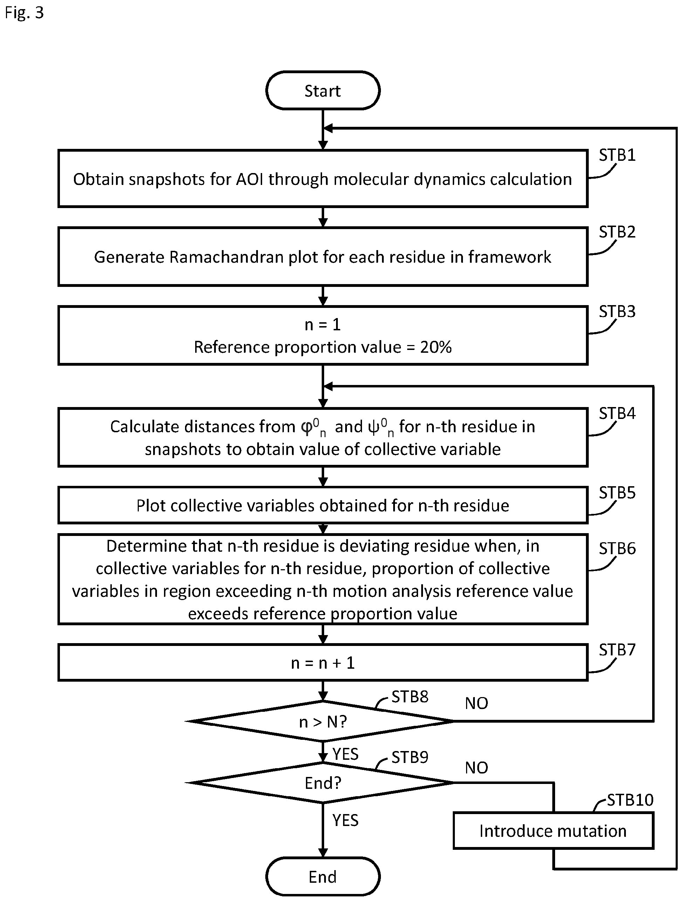

FIGs. 2 and 3 are each a flowchart of an example operation of the antibody dynamic structural property analyzer 1 in FIG. 1.

A process of analyzing reference antibodies will be described with reference to FIG. 2. In this example, the reference antibodies are medical antibodies. The input-output processing device 3 obtains identification data for the medical antibodies from a protein structure database and stores the data into the analysis target storage 11. The molecular movement calculator 29 performs, for example, molecular dynamics calculation to calculate the movements of at least atoms or molecules and obtain multiple snapshots for the medical antibodies stored in the analysis target storage 11 (step STA1). The snapshot storage 13 stores the obtained snapshots.

The protein structure database includes 17 types of medical antibodies. Although the 17 types of medical antibodies are calculated in the example described below, some of the antibodies or the antibodies including an additional medical antibody may be calculated in an embodiment of the present invention. Although the snapshots obtained in the example described below are 3000 frames per antibody, a different number of snapshots may be obtained in an embodiment of the present invention.

The number of residues in a framework region is N (N is a natural number). The R-plot processor 31 generates a Ramachandran plot for each residue of the N residues in the framework region and merges the data of all the antibodies (step STA2). The R-plot storage 15 stores the generated data. In one example, 3000 frames of snapshots per antibody are obtained for the total of 17 types of antibodies. The Ramachandran plot for each residue includes 51000 items (= 3000 frames × 17 antibodies) of data. In the embodiments of the present invention, N may be the number of some of the residues in the framework region or the total number of the residues in the framework region.

Amino acids are joined together with peptide bonds. The peptide bond (C-N) has partial double bond nature and cannot rotate freely in a peptide plane. However, bonds (an N-C_{α} bond and a C_{α}-C bond) involving an α carbon C_{α} (a carbon atom adjacent to the main chain and next to a focused functional group) are not rigid, allowing free rotation under a limit of the size and the nature of a side chain (R group) alone. The rotation angle about the N-C_{α} bond is referred to as φ (phi), and the rotation angle about the C_{α}-C bond is referred to as ψ (psi). The rotation angles φ and ψ are also referred to as twist angles or dihedral angles. The dihedral angles φ and ψ greatly determine the three-dimensional shape of the polypeptide backbone of a protein.

A Ramachandran plot includes the plots of ψ with respect to φ of an amino acid residue in a protein structure. Many software programs are available for Ramachandran plot generation. The R-plot processor 31 uses such a software program to generate a Ramachandran plot for each residue in the framework region.

The R-plot processor 31 merges data items of all the medical antibodies. In other words, the data items are integrated into a set of data based on a predetermined rule. In each snapshot, combinations of the dihedral angles φ and ψ are determined for respective residues. In the example in which 3000 frames of snapshots per antibody are obtained for the total of 17 types of medical antibodies, the Ramachandran plot for each residue includes 51000 combinations (= 3000 frames × 17 antibodies) of the dihedral angles φ and ψ.

The control processor 25 sets the initial value of the variable n to 1 (step STA3).

The R-plot analysis reference value determiner 33 uses the Ramachandran plot for the n-th residue to determine reference dihedral angle values φ⁰ₙ and ψ⁰ₙ (step STA4). The R-plot analysis reference value storage 17 stores the reference dihedral angle values φ⁰ₙ and ψ⁰ₙ. The reference dihedral angle values φ⁰ₙ and ψ⁰ₙ are, for example, modes in the Ramachandran plot for the n-th residue. A known method for finding and clustering a dense data group in a dataset is, for example, density-based spatial clustering of applications with noise (DBSCAN). Modes in a Ramachandran plot can be obtained using, for example, DBSCAN. In the embodiments of the present invention, the reference dihedral angle values φ⁰ₙ and ψ⁰ₙ may be values other than the modes.

The collective variable processor 35 calculates the distances from the reference dihedral angle values φ⁰ₙ and ψ⁰ₙ for the n-th residue to obtain a value of a collective variable for the dihedral angles φ and ψ of the n-th residue in each snapshot (step STA5). The collective variable storage 19 stores the calculated collective variable. The distances between the dihedral angles φ and ψ in the snapshot and the reference dihedral angle values φ⁰ₙ and ψ⁰ₙ may have smaller values for smaller differences and greater values for larger differences. The distances between the snapshots for the n-th residue and the reference dihedral angle values φ⁰ₙ and ψ⁰ₙ can be obtained using the distances between the dihedral angles φ and ψ of the n-th residue and the reference dihedral angle values φ⁰ₙ and ψ⁰ₙ for the n-th residue in each snapshot. The collective variable is closer to zero for highly common dynamic structural properties between antibodies and is a greater numerical value for less common dynamic structural properties. The two-dimensional data indicating the dihedral angles φ and ψ in the Ramachandran plot can thus be usable as one-dimensional data indicating a collective variable.

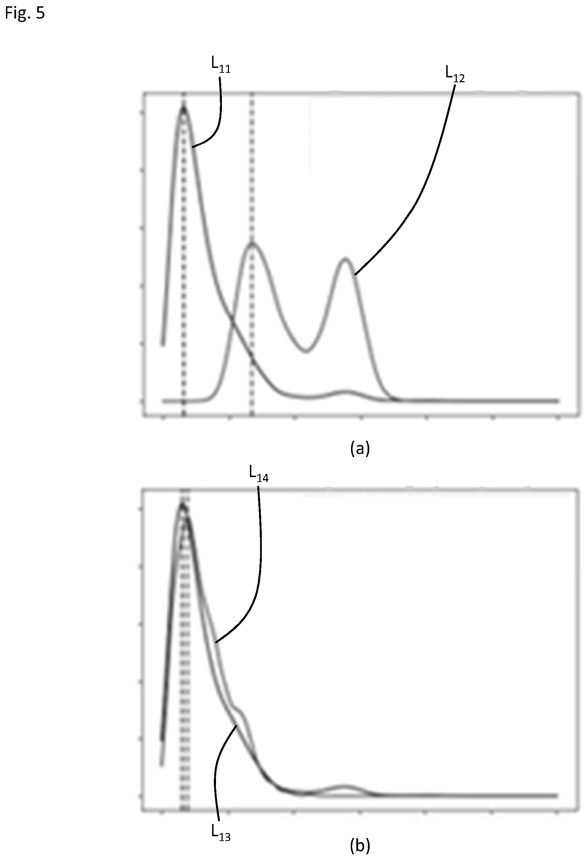

The collective variable plot processor 37 plots collective variables obtained for the n-th residue (step STA6). For example, when 3000 frames of snapshots per antibody are obtained for the total of 17 types of medical antibodies, the collective variables for each residue are 51000 items (= 3000 frames × 17 antibodies) of values. The collective variable plot storage 21 stores the plots of the collective variables. For example, the graphs in FIGs. 5a and 5b show a line L₁₁ and a line L₁₃ each representing the plots of the collective variables obtained with the medical antibodies.

The motion analysis storage 23 stores an n-th motion analysis reference value. The n-th motion analysis reference value for the n-th residue is used to define the n-th residue providing collective variables in a region less than or equal to the n-th motion analysis reference value as having common movements between antibodies. The motion analysis processor 39 may perform, as appropriate, processing for setting the n-th motion analysis reference value as a reference value for determining that the n-th residue has common movements between antibodies (step STA7). The n-th motion analysis reference value may be a value (e.g., 50) common to all the residues or may vary based on the distribution of the collective variables. For example, the motion analysis processor may adjust, for example, a formula of the collective variable for the n-th residue to obtain collective variables having a distribution based on the n-th motion analysis reference value, or may determine the n-th motion analysis reference value based on the distribution of the collective variables. This process may be eliminated unless the process is to be performed.

The control processor 25 increases n by one (step STA8). The control processor 25 determines whether n is greater than N (step STA9). When n is greater than N, the process in FIG. 2 ends. When n is not greater than N, the processing returns to step STA4.

A process for analyzing a target antibody (antibody of interest or AOI) will be described with reference to FIG. 3.

The input-output processing device 3 obtains identification data for a target antibody (AOI) without mutation from, for example, an analyzer that analyzes an actual antibody to obtain identification data for the antibody or a simulator that generates identification data for the antibody through simulation, and stores the data into the analysis target storage 11. The molecular movement calculator 29 performs, for example, molecular dynamics calculation to calculate the movements of at least atoms or molecules and obtain multiple snapshots for the target antibody stored in the analysis target storage 11 (step STB1). The snapshot storage 13 stores the obtained snapshots. For example, 3000 frames of snapshots are obtained for the AOI.

The R-plot processor 31 generates a Ramachandran plot for each residue of the N residues in the framework region of the AOI and obtains dihedral angles φ and ψ (step STB2). The R-plot storage 15 stores the obtained Ramachandran plot. For example, when 3000 frames of snapshots are obtained for the AOI, the Ramachandran plot for each residue includes 3000 combinations of the dihedral angles φ and ψ.

The control processor 25 sets the initial value of the variable n to 1 and sets the reference proportion value to 20% (step STB3).

The collective variable processor 35 calculates, for the n-th residue, the distances from the reference dihedral angle values φ⁰ₙ and ψ⁰ₙ for the n-th residue to obtain a value of a collective variable for the dihedral angles φ and ψ of the n-th residue in each snapshot (step STB4). For example, when 3000 frames of snapshots are obtained for the AOI, collective variables for each residue are 3000 items of values. The collective variable storage 19 stores the calculated values of the collective variables.

The collective variable plot processor 37 plots the collective variables obtained for the n-th residue (step STB5). The collective variable plot storage 21 stores the plots of the collective variables. For example, the graph in FIG. 5a shows a line L₁₂ representing the plots of the collective variables for the antibody before introduction of a mutation. The graph in FIG. 5b shows a line L₁₄ representing the plots of the collective variables for the antibody after introduction of a mutation.

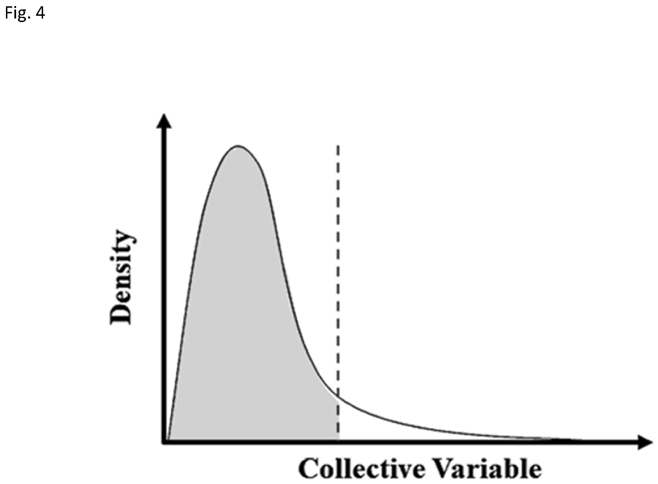

The motion analysis processor 39 determines that the n-th residue is a deviating residue when, in the collective variables for the n-th residue, the proportion of collective variables in a region exceeding the n-th motion analysis reference value exceeds the reference proportion value (step STB6). The motion analysis processor 39 does not determine that the n-th residue is a deviating residue when, in the collective variables for the n-th residue, the proportion of collective variables in a region exceeding the n-th motion analysis reference value does not exceed the reference proportion value. The motion analysis storage 23 stores information as to whether the n-th residue is determined as a deviating residue. FIG. 4 is a graph of example collective variables for the n-th residue in which the proportion of collective variables in a region exceeding an n-th motion analysis reference value does not exceed a reference proportion value. The horizontal axis indicates the value of the collective variable, and the vertical axis indicates the density (the number of snapshots for the value of each collective variable divided by the total number of snapshots).

The control processor 25 increases n by one (step STB7). The control processor 25 determines whether n is greater than N (step STB8). When n is not greater than N, the processing returns to step STB4.

When n is greater than N, the control processor 25 causes, for example, the input-output processing device 3 to display the residue determined as a deviating residue and determines whether the process is to end (step STB9). For example, when the user concludes that the target antibody is stabilized and provides an instruction to end the process with the input-output processing device 3, the process in FIG. 3 ends.

When the user provides an instruction to continue the process with the input-output processing device 3, the process does not end. In this case, data used for mutation introduction (e.g., data specifying an amino acid for introduction of a mutation and identifying the type of the mutation to be introduced) is input into the input-output processing device 3. Framework regions have highly conserved amino acid residues and structures between antibodies and have similar dynamic structural properties. Structural distortion occurs around a region deviating from a common movement. Introducing a mutation to correct the distortion may produce an antibody with high thermal stability. A candidate mutation site is located spatially around the deviating residue. The user thus introduces a mutation around the residue determined as a deviating residue to stabilize the antibody. Determining a deviating residue in step STB6 is useful for determining the site for introduction of a mutation. The mutation processor 27 introduces a mutation to the specified amino acid in the target antibody in the analysis target storage 11 and newly stores the changed antibody into the analysis target storage 11 (step STB10). The processing returns to step STB1.

An example experiment conducted by the inventors using trastuzumab antibodies will now be described with reference to FIGs. 5a to 8h. In the graph in each figure, the horizontal axis indicates the value of the collective variable, and the vertical axis indicates the density.

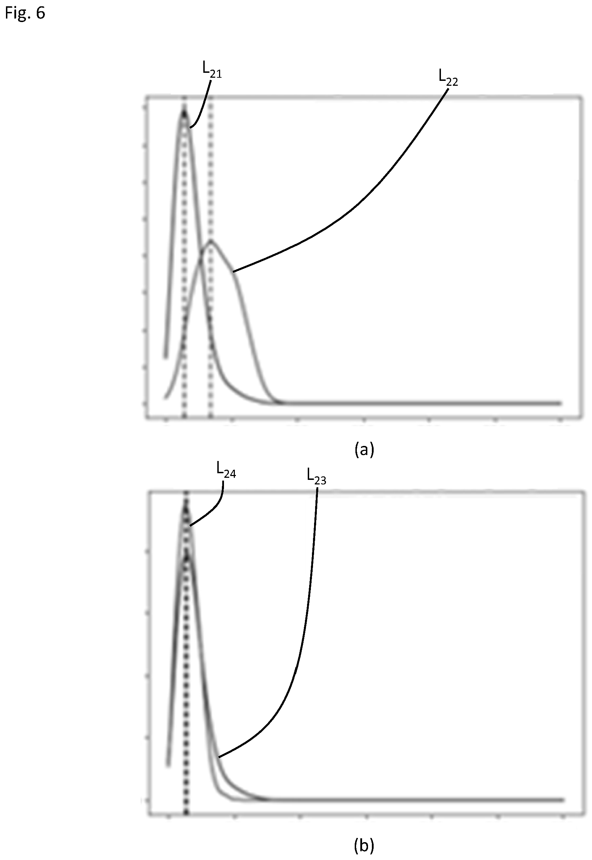

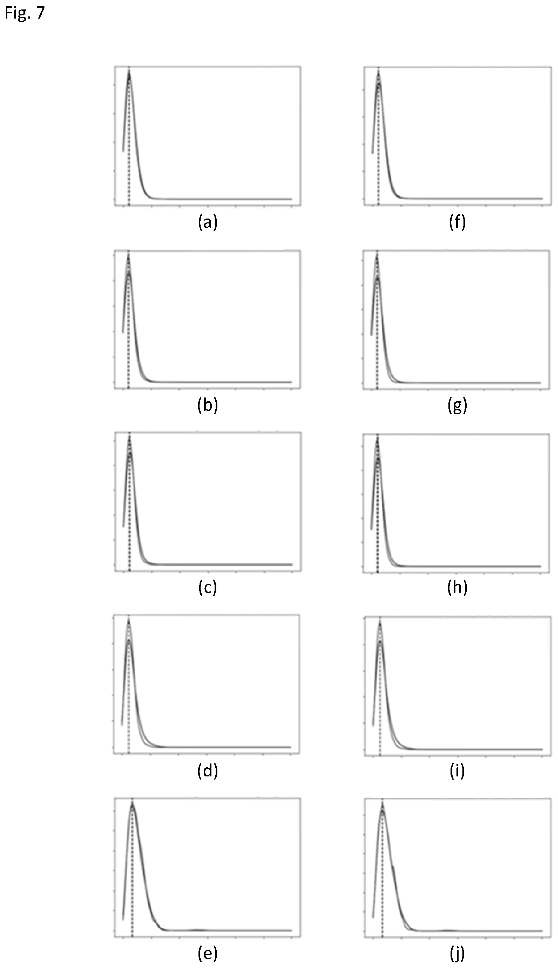

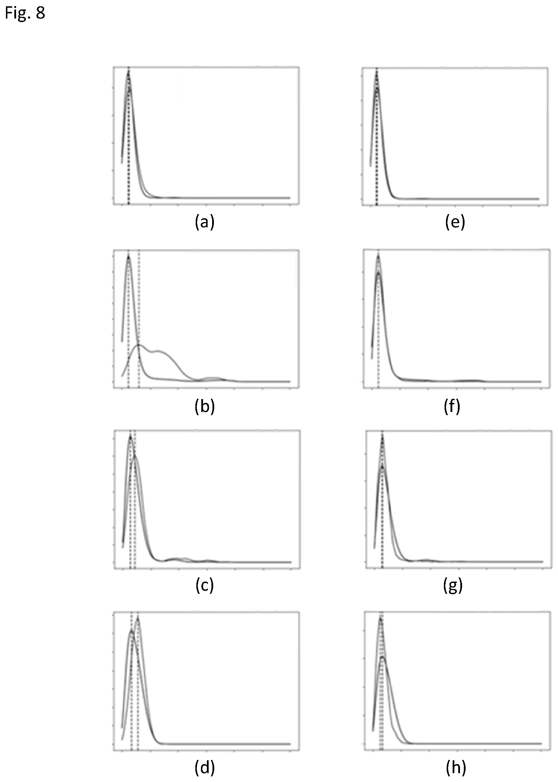

FIGs. 5a, 6a, 7a to 7e, and 8a to 8d show the plots of collective variables obtained with an antibody (wild type) before introduction of a mutation. FIG. 5a shows the plots for the 14th residue. FIG. 6a shows the plots for the 18th residue. FIGs. 7a to 7e show the plots for the 8th to 12th residues. FIGs. 8a to 8d show the plots for the 13th, 15th, 16th, and 17th residues.

FIGs. 5b, 6b, 7f to 7j, and 8e to 8h show the plots of collective variables obtained with an antibody after introduction of a mutation. FIG. 5b shows the plots for the 14th residue. FIG. 6b shows the plots for the 18th residue. FIGs. 7f to 7j show the plots for the 8th to 12th residues. FIGs. 8e to 8h show the plots for 13th, 15th, 16th, and 17th residues.

Referring to FIG. 5a for the 14th residue, the line L₁₁ represents the plots of the collective variables obtained with the medical antibodies, and the line L₁₂ represents the plots of the collective variables obtained with the wild type antibody before introduction of a mutation.

The line L₁₂ includes more collective variables having greater values than the line L₁₁. The motion analysis processor 39 determines that the 14th residue is a deviating residue because, in the collective variables for the 14th residue, the proportion of collective variables in a region exceeding the 14th motion analysis reference value exceeds the reference proportion value. The user introduces a mutation around the 14th residue.

Referring to FIG. 5b, the line L₁₃ represents the plots of the collective variables obtained with the medical antibodies, and is the same as the line L₁₁ in FIG. 5a. The line L₁₄ represents the plots of the collective variables obtained with the antibody after introducing a mutation around the 14th residue.

The line L₁₄ and the line L₁₃ have substantially the same peak values (dotted lines). In FIG. 5b, the proportion of collective variables in a region exceeding the 14th motion analysis reference value to the collective variables for the 14th residue does not exceed the reference proportion value. The motion analysis processor 39 thus does not determine the 14th residue as a deviating residue.

Referring to FIG. 6a for the 18th residue, a line L₂₁ represents the plots of the collective variables obtained with the medical antibodies, and a line L₂₂ represents the plots of the collective variables obtained with the wild type antibody before introduction of a mutation. The line L₂₂ includes more collective variables with greater values than the line L₂₁.

Referring to FIG. 6b, a line L₂₃ represents the plots of the collective variables obtained with the medical antibodies, and a line L₂₄ represents the plots of the collective variables obtained with the antibody after introduction of a mutation around the 14th residue. FIG. 6b shows that the mutation introduced around the 14th residue has also stabilized the 18th residue.

FIGs. 7a to 7e and 8a to 8d are compared with FIGs. 7f to 7j and 8e to 8h. These figures show that the distributions of the collective variables have no major change before and after the mutation is introduced.

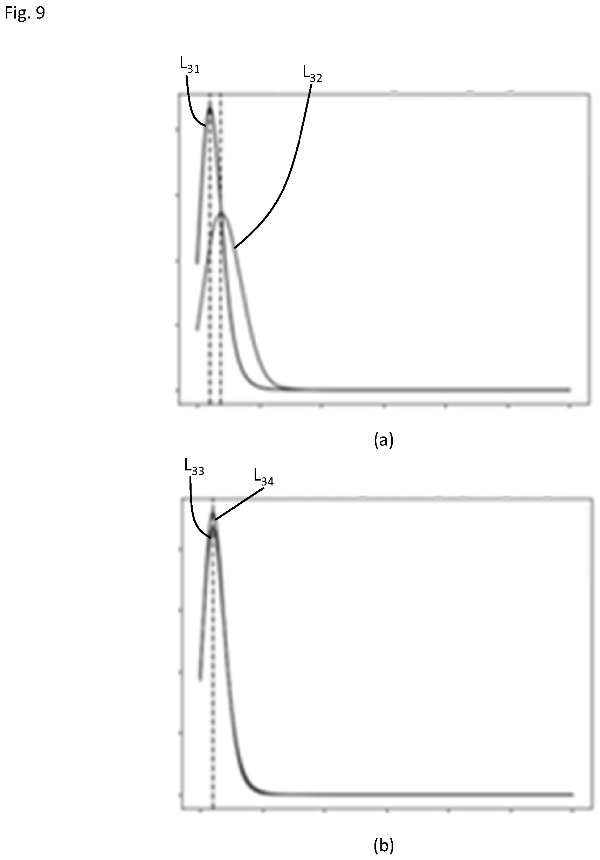

An example experiment conducted by the inventors using OKT3 antibodies will be described with reference to FIGs. 9a to 12j. In the graph in each figure, the horizontal axis indicates the value of the collective variable, and the vertical axis indicates the density.

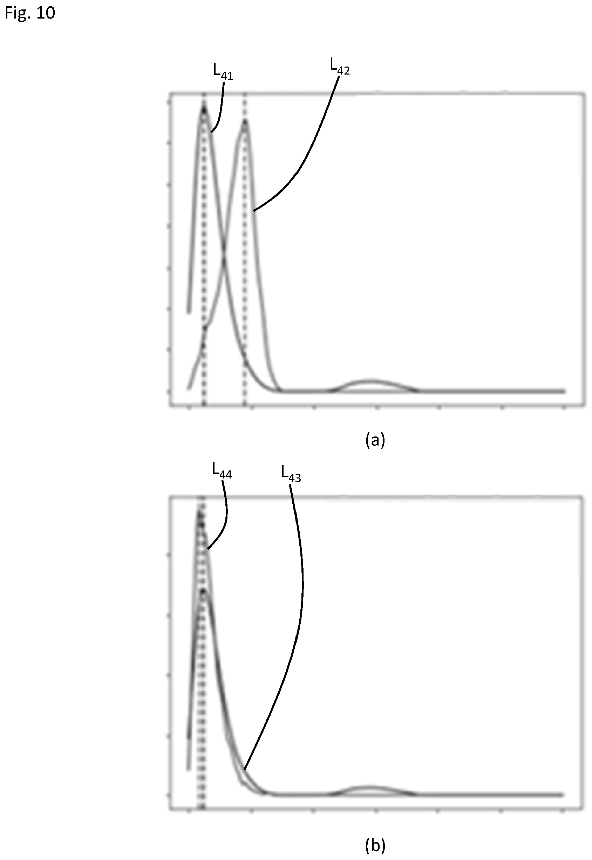

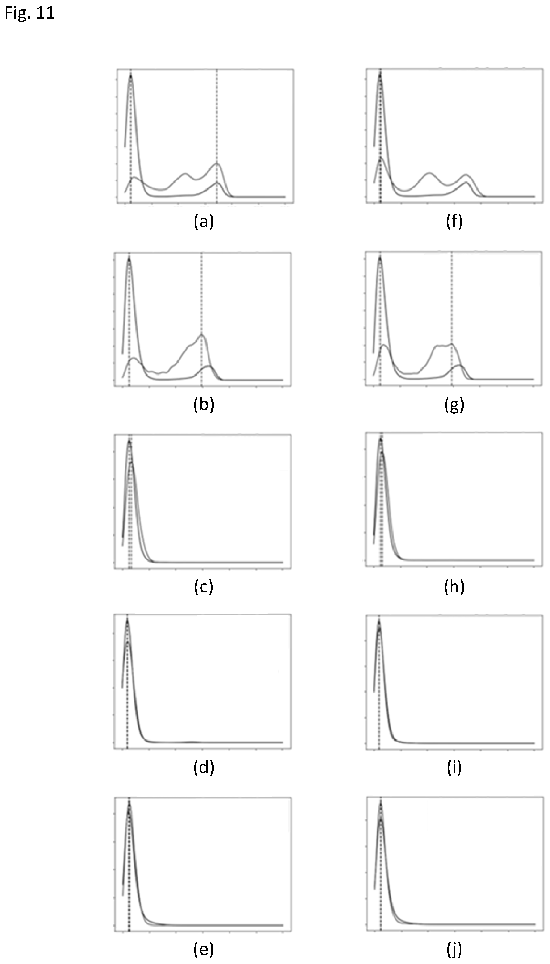

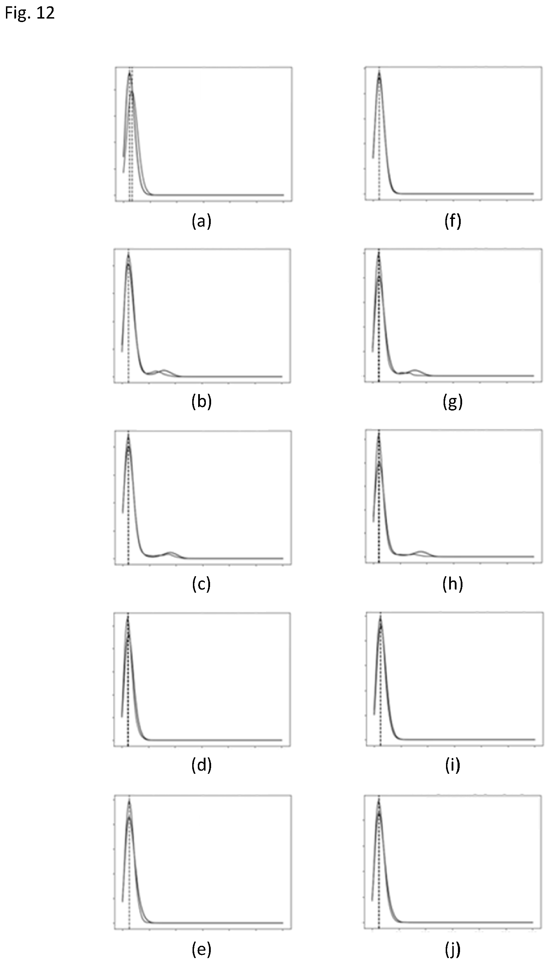

FIGs. 9a, 10a, 11a to 11e, and 12a to 12e show the plots of collective variables obtained with an antibody (wild type) before introduction of a mutation. FIG. 9a shows the plots for the 9th residue. FIG. 10a shows the plots for the 27th residue. FIGs. 11a to 11e show the plots for the 24th, 25th, 26th, 28th, and 29th residues. FIGs. 12a to 12e show the plots for the 30th to 34th residues.

FIGs. 9b, 10b, 11f to 11j, and 12f to 12j show the plots of collective variables obtained with the antibody after introduction of a mutation. FIG. 9b shows the plots for the 9th residue. FIG. 10b shows the plots for the 27th residue. FIGs. 11f to 11j show the plots for the 24th, 25th, 26th, 28th, and 29th residues. FIGs. 12f to 12j show the plots for the 30th to 34th residues.

Referring to FIG. 9a for the 9th residue, a line L₃₁ represents the plots of the collective variables obtained with the medical antibodies, and a line L₃₂ represents the plots of the collective variables obtained with the wild type antibody before introduction of a mutation.

The line L₃₂ includes more collective variables having greater values than the line L₃₁. The motion analysis processor 39 thus determines that the 9th residue is a deviating residue because, in the collective variables for the 9th residue, the proportion of collective variables in a region exceeding the 9th motion analysis reference value exceeds the reference proportion value. The user introduces a mutation around the 9th residue.

Referring to FIG. 9b, a line L₃₃ represents the plots of the collective variables obtained with the medical antibodies, and a line L₃₄ represents the plots of the collective variables obtained with the antibody after introduction of a mutation around the 9th residue.

The line L₃₄ and the line L₃₃ have substantially the same peak values (dotted lines). In FIG. 9b, the proportion of collective variables in a region exceeding the 9th motion analysis reference value to the collective variables for the 9th residue does not exceed the reference proportion value. The motion analysis processor 39 thus does not determine that the 9th residue is a deviating residue.

Referring to FIG. 10a for the 27th residue, a line L₄₁ represents the plots of the collective variables obtained with the medical antibodies, and a line L₄₂ represents the plots of the collective variables obtained with the wild type antibody before introduction of a mutation. The line L₄₂ includes more collective variables with greater values than the line L₄₁.

Referring to FIG. 10b, a line L₄₃ represents the plots of the collective variables obtained with the medical antibodies, and a line L₄₄ represents the plots of the collective variables obtained with the antibody after introduction of a mutation around the 9th residue. FIG. 10b shows that the mutation introduced around the 9th residue also stabilizes the 27th residue.

FIGs. 11a to 11e and 12a to 12e are compared with FIGs. 11f to 11j and 12f to 12j. These figures show that the distributions of the collective variables have no major change before and after the mutation is introduced.

Antibodies are used in pharmaceuticals, diagnostic agents, and sensor elements. However, antibodies are susceptible to physical stress (e.g., heat, friction, or vibration). Stable antibodies are to be produced for practical use. Analysis using RMSF values relies on manual analysis and thus has a limit in the number of searchable variants. Additionally, residue movements are averaged in RMSF values. Rare movements or small movements are thus not easily evaluated.

Framework regions have highly conserved amino acid residues and structures between antibodies and have similar dynamic structural properties. Structural distortion may occur around a region deviating from standard dynamic structural properties, possibly reducing stability. The inventors used a molecular dynamics method to computationally simulate the movements of a protein and performed molecular dynamics calculation for the medical antibodies (17 types in total) registered with the protein structure database. The obtained results underwent clustering analysis, and dynamic structural properties common to the antibodies were extracted. Collective variables were defined, based on this data, to be closer to zero for highly common dynamic structural properties between antibodies and to have greater numerical values for less common dynamic structural properties. A normal value was defined when the proportion of frames providing collective variables having values greater than a threshold was less than or equal to the reference proportion value. An abnormal value was defined when the proportion was greater than the reference proportion value. A mutation was computationally introduced around the region providing an abnormal value. Molecular dynamics calculation was performed again to calculate the collective variables. The results revealed that the mutant with improved thermal stability had no abnormal value. The inventors examined multiple antibodies and confirmed that the method is versatile.

The technique according to the embodiment of the present invention achieves analysis without averaging that can find a locally denatured region unfindable with analysis using RMSF values. Multiple antibodies can also be compared at the same time. The technique according to the embodiment of the present invention allows rapid designing of antibodies with notably high evaluation performance and high thermal stability. The technique according to the embodiment of the present invention can shorten the time taken for obtaining stable antibodies to about two weeks from conventional several months to about one year. Additionally, more stable antibodies may be produced.

### REFERENCE SIGNS LIST

- 1: antibody dynamic structural property analyzer
- 3: input-output processing device
- 5: storage device
- 7: processing device
- 11: analysis target storage
- 13: snapshot storage
- 15: R-plot storage
- 17: R-plot analysis reference value storage
- 19: collective variable storage
- 21: collective variable plot storage
- 23: motion analysis storage
- 25: control processor
- 27: mutation processor
- 29: molecular movement calculator
- 31: R-plot processor
- 33: R-plot analysis reference value determiner
- 35: collective variable processor
- 37: collective variable plot processor
- 39: motion analysis processor

## Claims

1. An antibody dynamic structural property analyzer for analyzing dynamic structural properties of a target antibody using identification data for the target antibody stored in an analysis target storage, the analyzer comprising:
a molecular movement calculator configured to calculate movements of at least atoms or molecules and obtain a plurality of snapshots for the target antibody;
an R-plot processor configured to generate a Ramachandran plot for an n-th residue in N residues of the target antibody and obtain dihedral angles φ and ψ of the n-th residue in each of the plurality of snapshots, N being a natural number, n being a natural number from 1 to N;
a collective variable processor configured to calculate distances from reference dihedral angle values φ⁰ₙ and ψ⁰ₙ for the n-th residue to obtain a value of a collective variable for the dihedral angles φ and ψ of the n-th residue in each of the plurality of snapshots; and
a motion analysis processor configured to determine that the n-th residue is a deviating residue when, in collective variables for the n-th residue in the plurality of snapshots, a proportion of collective variables having values in a region exceeding a motion analysis reference value exceeds a reference proportion value.

2. The antibody dynamic structural property analyzer according to claim 1, further comprising:
a mutation processor configured to store, into the analysis target storage, identification data for another target antibody resulting from introduction of a mutation into the target antibody.

3. The antibody dynamic structural property analyzer according to claim 1, further comprising:
an R-plot analysis reference value determiner,
wherein the molecular movement calculator calculates movements of at least atoms or molecules and obtains a plurality of snapshots for a plurality of reference antibodies,
the R-plot processor generates a Ramachandran plot for n-th residues of the plurality of reference antibodies and obtains dihedral angles φ and ψ of each of the n-th residues in the plurality of snapshots, and
the R-plot analysis reference value determiner determines the reference dihedral angle values φ⁰ₙ and ψ⁰ₙ for the n-th residue using the Ramachandran plot for the n-th residues of the plurality of reference antibodies.

4. An antibody dynamic structural property analysis method for analyzing dynamic structural properties of a target antibody using identification data for the target antibody stored in an analysis target storage, the method comprising:
calculating, with a molecular movement calculator included in an information processing device, movements of at least atoms or molecules and obtaining a plurality of snapshots for the target antibody;
generating, with an R-plot processor included in the information processing device, a Ramachandran plot for an n-th residue in N residues of the target antibody and obtaining dihedral angles φ and ψ for the n-th residue, N being a natural number, n being a natural number from 1 to N;
calculating, with a collective variable processor included in the information processing device, distances from reference dihedral angle values φ⁰ₙ and ψ⁰ₙ to obtain a value of a collective variable for the dihedral angles φ and ψ of the n-th residue in each of the plurality of snapshots; and
determining, with a motion analysis processor included in the information processing device, that the n-th residue is a deviating residue when, in collective variables for the n-th residue in the plurality of snapshots, a proportion of collective variables having values in a region exceeding a motion analysis reference value exceeds a reference proportion value.

5. The method according to claim 4, further comprising:
storing, into the analysis target storage, with a mutation processor included in the information processing device, identification data for another target antibody resulting from introduction of a mutation into the target antibody.

6. The method according to claim 4, further comprising:
calculating, with the molecular movement calculator, movements of at least atoms or molecules and obtaining a plurality of snapshots for a plurality of reference antibodies;
generating, with the R-plot processor, a Ramachandran plot for n-th residues of the plurality of reference antibodies and obtaining dihedral angles φ and ψ of each of the n-th residues in the plurality of snapshots; and
determining, with an R-plot analysis reference value determiner included in the information processing device, the reference dihedral angle values φ⁰ₙ and ψ⁰ₙ for the n-th residue using the Ramachandran plot for the n-th residues of the plurality of reference antibodies.

7. A program for causing a computer to function as the antibody dynamic structural property analyzer according to claim 1.
